# FASCICULE DE BREVET EUROPEEN

(11) **EP 3 612 636 B1**
(45) Date de publication et mention de la délivrance du brevet: **02.06.2021**
(21) Numéro de dépôt: 18719811.4
(22) Date de dépôt: 18.04.2018
(51) Int. Cl.: C12P 7/56, C12M 1/00

(54) **PROCÉDÉ DE PRODUCTION SIMULTANÉE D'ACIDE LACTIQUE ET D'ALCOOL OU BIOGAZ AU DÉPART DE CÉRÉALES**
VERFAHREN ZUR SIMULTANEN HERSTELLUNG VON MILCHSÄURE UND ALKOHOL ODER BIOGAS AUS GETREIDE
PROCESS FOR THE SIMULTANEOUS PRODUCTION OF LACTIC ACID AND ALCOHOL OR BIOGAS FROM CEREALS

(30) Priorité: 20.04.2017 BE 201705275
(43) Date de publication de la demande: 26.02.2020
(73) Titulaire: Galactic S.A., 7760 Escanaffles (BE)
(72) Inventeur: VAN GANSBERGHE, Frédéric, 7760 Escanaffles (BE); COSZACH, Philippe, 7760 Escanaffles (BE)
(74) Mandataire: Pronovem
(86) Numéro de dépôt international: PCT/EP2018/059861
(87) Numéro de publication internationale: WO 2018/192952

(56) Documents cités:
- WO-A2-2013/148207
- US-A- 2 588 460
- GYENGE L ET AL: "Efficiency of biogas production from corn bioe-thanol by-products using different inocula", 2013 4TH INTERNATIONAL YOUTH CONFERENCE ON ENERGY (IYCE), IEEE, 6 juin 2013 (2013-06-06), pages 1-6, XP032484976, DOI: 10.1109/IYCE.2013.6604171 [extrait le 2013-09-18]
- JOHN R P ET AL: "Direct lactic acid fermentation: Focus on simultaneous saccharification and lactic acid production", BIOTECHNOLOGY ADVANCES, ELSEVIER PUBLISHING, BARKING, GB, vol. 27, no. 2, 1 mars 2009 (2009-03-01), pages 145-152, XP025873360, ISSN: 0734-9750, DOI: 10.1016/J.BIOTECHADV.2008.10.004 [extrait le 2008-10-31]

## Description

### Domaine de l'invention

La présente invention se rapporte à un procédé en continu de production simultanée d'acide lactique (comme premier produit de fermentation) et d'un second produit de fermentation à partir de céréales et en particulier de grains de maïs.

Le procédé de la présente invention comprend plusieurs étapes dont les principales sont le broyage à sec des épis de maïs, ce qui constituera le flux principal, la séparation des déchets et autres produits difficilement fermentables de ce broyage, qui constitueront le flux secondaire et qui seront traités pour produire de l'alcool et/ou du biogaz, tandis que le broyat initial (épuré des déchets et autres produits difficilement fermentables) est traité séparément pour être ensuite fermenté dans un fermenteur pour produire de l'acide lactique.

Le procédé de l'invention a également pour objet de maximiser la valorisation économique des grains de maïs.

### État de la technique

Suite à la libéralisation du marché du sucre, mais également des barrières à l'entrée mises en place au niveau de l'Europe, une tendance nette à la hausse apparait au niveau des prix des matières premières utilisées dans les processus de production de composés biochimiques de commodités comme les biocarburants (ex bioéthanol), l'acide citrique, l'acide succinique, l'acide lactique,.... Dès lors, il devient impératif pour ces acteurs industriels d'optimiser leurs voies d'approvisionnement afin de disposer d'une matière première disponible en grande quantité, à un coût compétitif et avec une qualité suffisante que pour permettre la viabilité du processus biotechnologique sans pour autant nécessiter des adaptations importantes des unités de production déjà existantes. Dans ce cadre, plusieurs matières premières, comme par exemple le maïs, le blé, l'orge, le sorgho, le riz, le seigle ou l'avoine, sont envisageables, et plus particulièrement le maïs peut répondre favorablement à plusieurs de ces critères.

Dans l'état de la technique qui se rapporte à ces processus de traitement des céréales, on sait que celles-ci, en ce compris les épis de maïs, peuvent être traités par broyage humide ou par broyage à sec, et que le broyage à sec est généralement le plus simple et le moins onéreux des deux voies d'exploitation.

Il est également de pratique courante de séparer, à l'issue du broyage ou plus tard dans la conduite du procédé, les déchets comprenant les fibres, les germes et autres produits difficilement fermentables du courant initial qui est constitué de produits directement fermentables.

Ceci est d'ailleurs décrit dans le brevet EP3121258 qui mentionne la nécessité de séparer les éléments non désirables ainsi que les difficilement fermentables avant la fermentation pour la production de biofuel, mais préconise également de ne pas se soucier de la fraction séparée, car son traitement requiert des investissements conséquents, ce qui ne permet pas une bonne valorisation économique du procédé.

A l'heure actuelle, il n'existe pas de procédé simple et peu onéreux pour produire à la fois de l'acide lactique (comme premier produit de fermentation) et un second produit de fermentation comme un alcool et/ou du biogaz au départ de grains céréaliers.

Or l'acide lactique est un produit qui rencontre chaque jour de nouvelles applications, notamment en tant que conservateur alimentaire, mais également dans la synthèse de solvants ou dans les polymères biodégradables. D'autre part, après valorisation sous forme d'éthanol, la fraction résiduelle du flux secondaire peut également être valorisée pour la préparation d'aliments pour bétail ou sous forme d'autres applications, comme par exemple le biogaz.

Il apparaît donc qu'il existe un besoin réel pour un procédé simple et peu onéreux pour produire à la fois de l'acide lactique et un second produit de fermentation au départ de céréales, et plus particulièrement au départ de grains ou d'épis de maïs.

### Buts de l'invention

Le procédé de la présente invention a pour objet de permettre la production simultanée de l'acide lactique, comme premier produit de fermentation, et d'un second produit de fermentation, au départ de céréales comme le maïs.

Le procédé de la présente invention a pour objet de permettre la production simultanée de l'acide lactique et d'un alcool comme l'éthanol ou le butanol au départ de céréales comme les grains ou les épis de maïs.

Le procédé de la présente invention a pour objet de permettre la production simultanée de l'acide lactique et de biogaz au départ de céréales comme les grains ou les épis de maïs.

Un autre objet du procédé de l'invention est de valoriser la partie comprenant les éléments non désirables et/ou difficilement fermentables pour les transformer en alcool ou en biogaz.

Un autre objet de l'invention est de maximiser la valorisation économique du traitement des céréales, en particulier des grains ou des épis de maïs.

La Demanderesse a maintenant trouvé que l'on pouvait rencontrer les buts décrits ci-dessus en opérant un procédé qui comprend les étapes suivantes :
- Broyer à sec dans un broyeur 100 les grains et/ou les épis de maïs 20;
- Récupérer le flux 131 résultant du broyage et le mélanger dans un mélangeur 102 avec un courant d'eau (c.-à-d. effectuer une étape de liquéfaction) afin de créer un slurry 103;
- Soumettre le slurry 103 à une étape de saccharification 104 dans un réacteur et produire un flux de dextrose brut 105;
- Séparer le courant de dextrose brut 105 en deux flux, le premier flux étant constitué d'un flux de dextrose liquide 108 et le second flux de dextrose comprenant la partie solide 21 constituée de deux flux résultant de deux étapes successives de séparation (en utilisant successivement deux séparateurs 106 et 107) et formant finalement le flux 21;
- Séparer dans un séparateur 109 l'huile résultante du broyage et se trouvant dans le flux de dextrose liquide 108 et d'une part récupérer cette huile pour traitement ultérieur (comme une étape d'affinage huiles 130) et d'autre part récupérer un flux de dextrose purifié 110;
Caractérisé en ce que l'on :
- Soumet le flux de dextrose purifié 110 à une étape de séparation sélective des sucres 111 et récupérer un flux de dextrose purifié 116, qui est plus pur que le flux 110, avant de le soumettre à une fermentation 118, et récupérer d'autre part un flux riche en oligosaccharides 29;
- Soumet le flux de dextrose purifié 116 à une fermentation 118 en présence d'un microorganisme 26 approprié pour produire de l'acide lactique 28;
- Récupère le flux de dextrose solide 21, résultant de deux étapes successives de séparation (en utilisant successivement deux séparateurs 106 et 107) via une conduite 121 contenant les produits difficilement fermentables, ainsi qu'un flux riche en oligosaccharides 29, qui résulte de la séparation sélective des sucres 111 via une conduite 112, pour les hydrolyser dans un réacteur 113 dans des conditions d'hydrolyse et les transformer en un flux de dextrose fermentable 114;
- Soumet le flux de dextrose fermentable 114 à une fermentation dans un fermenteur 120 pour produire un second produit de fermentation 101, de préférence choisi parmi le groupe constitué par l'éthanol, le butanol, et/ou du biogaz.

Il est bien entendu que comme céréales on comprend aussi bien le maïs que le blé, l'orge, le sorgho, le seigle, le riz, l'avoine, ou autres similaires.

### Principaux éléments caractéristiques de l'invention

Le procédé de la présente invention comprend un broyage à sec, dans un broyeur approprié 100, de céréales 20, en l'occurrence des grains et/ou des épis de maïs, pour produire simultanément deux flux distincts de sucre ou dextrose.

Ce broyage s'effectue nécessairement par voie sèche et non par voie humide comme c'est généralement le cas pour les procédés des glucoseries.

Après avoir broyé à sec dans le broyeur 100 les grains et/ou les épis de maïs 20, on récupère le broyat 131 que l'on mélange avec un courant d'eau dans le mélangeur 102 dans lequel on réalise une étape de liquéfaction (hydrolyse de l'amidon en maltodextrines et oligosaccharides) pour former un slurry 103, suivie d'une étape de saccharification 104 (transformation des sucres complexes en sucres simples comme le glucose).

Après l'étape de saccharification 104, on sépare le flux de dextrose en deux parties, l'une liquide 108 qui constitue le flux principal et contient le flux de dextrose, avec une teneur en dextrose DE (Dextrose = glucose Equivalent) de l'ordre de 80% et une seconde partie solide 21 constituée du flux secondaire qui comprend les éléments difficilement fermentables.

Ce flux secondaire 21 comprend notamment les éléments fibreux, les germes et des protéines.

Selon un mode d'exécution du procédé de l'invention, on traite ensuite le flux principal 108 pour enlever l'huile, via le flux 25, qui résulte du broyage des grains de maïs; après cette première purification, le flux principal 110 est soumis à une étape de filtration 115 et on en sépare les oligosaccharides via le flux 29, afin d'avoir un flux de dextrose relativement pur 116 (DX (pourcentage de glucose)>95% voire > 99%) pour être soumis à la fermentation 118 en présence d'un microorganisme 26 de type bactérie, levure ou moisissure pour produire de l'acide lactique 28.

Toujours selon un mode d'exécution du procédé de l'invention, on récupère le flux secondaire 21 et on y ajoute les oligosaccharides 29 récupérés du flux principal 108 pour l'envoyer à une étape d'hydrolyse dans un réacteur 113 en présence d'un acide fort. On dirige le flux résultant 114 vers un second fermenteur 120 afin de transformer ce second flux de dextrose en alcool, pour produire du bioéthanol, ou du butanol, et/ou en biogaz.

### Brève description des figures

Le procédé de la présente invention est également décrit au moyen de la figure suivante:

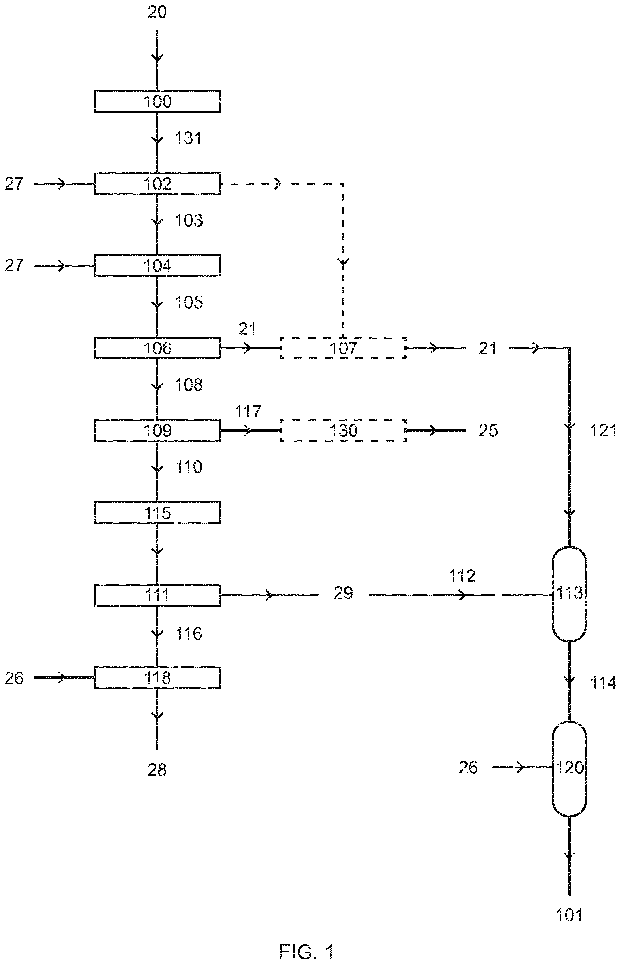

La Figure 1 représente un diagramme schématique (d'un mode d'exécution) du procédé de l'invention partant du broyage à sec dans le broyeur 100 des épis de maïs 20 pour produire deux flux de dextrose, l'un destiné à produire comme premier produit de fermentation de l'acide lactique 28, l'autre destiné à produire un second produit de fermentation 101 comme un alcool ou du biogaz.

### Description détaillée de l'invention

Selon la Figure 1, on décrit un système de broyage à sec dans un broyeur 100 des épis de maïs 20 pour produire en final deux (flux ou) courants de dextrose comme cela sera décrit ci-dessous.

Après passage au broyage à sec dans un broyeur 100, la farine de maïs ainsi obtenue ainsi que le reste 131 du broyat comprenant les fibres, les germes, les éléments difficilement fermentables, sont ensuite introduits dans le mélangeur 102, où ils sont mélangés avec un courant d'eau fraiche et un courant d'eau de recyclage d'une étape ultérieure du procédé; on peut également ajouter dans ce mélangeur 102 une enzyme 27, comme l'alpha-amylase; on peut si nécessaire chauffer le slurry obtenu 103 à une température comprise entre 60 et 100°C pendant une période de 30 à 120 minutes; on obtient ainsi le slurry 103.

Ce slurry 103, qui contient les grains de maïs, les germes, les fibres, les protéines ainsi que l'huile qui résulte du broyage des grains de maïs, est alors envoyé à l'étape de saccharification 104. La teneur en matière solide sèche de ce slurry 103 représente environ 25 à 40% en poids.

L'étape de saccharification 104 produit un flux de dextrose brut 105.

Selon un mode d'exécution du procédé de l'invention, on effectue la réaction de saccharification 104 en deux étapes.

Lors de la première étape, on ajuste d'abord le pH entre 3,5 et 7,0 tout en maintenant la température entre l'ambiance et 100°C pendant une période de 1 à 6 heures, afin de transformer l'amidon insoluble en dextrose. On peut également ajouter un catalyseur pour effectuer la réaction comme de l'alpha-amylase à raison de 0,01 à 0,1% en poids.

Lors de la seconde étape, on réajuste le pH à une valeur comprise entre 4,0 et 5,0 tout en chauffant à une température allant de l'ambiance à 180°C, pendant une période de 2 à 5 heures, toujours pour parfaire la conversion de l'amidon insoluble en dextrose. Dans cette étape également on peut ajouter un catalyseur comme la glucoamylase ou l'alpha-amylase à raison de 0,01 à 0,2% en poids.

Le flux de dextrose 105 ainsi récupéré a une teneur en dextrose d'environ 90DE.

Après l'étape de saccharification 104, on effectue une première séparation dans le flux de dextrose brut 105 en le faisant passer sur un filtre 106 qui va séparer la partie liquide 108 de la partie solide 21, cette dernière comprend les fibres, les germes et les grits ainsi que les éléments difficilement fermentables.

Cette partie solide 21 est alors envoyée via la conduite 121 vers un réacteur 113 afin d'être traitée pour être transformée en un (flux ou) courant de dextrose fermentable 114.

D'autre part, la partie liquide 108 issue du séparateur est envoyée vers un autre séparateur 109 afin d'enlever l'huile provenant du broyage des grains de maïs, la séparation de cette huile est effectuée par une méthode bien connue de l'homme de l'art.

L'huile extraite est récupérée via la conduite 117 pour être purifiée et utilisée à d'autres fins.

Le flux de dextrose 110, pratiquement exempt d'huile, est éventuellement filtré 115 une nouvelle fois afin d'éliminer toute particule solide avant d'être envoyé dans un réacteur pour enlever les oligosaccharides 29 et obtenir un flux de dextrose 116 aussi pur que possible avant d'être soumis à la fermentation 118.

Selon un mode d'exécution préféré du procédé de l'invention afin d'avoir une fermentation aisée, une productivité élevée et un profil d'impureté plus facilement gérable au niveau de la production d'acide lactique 28, il est préférable d'augmenter la pureté du flux de dextrose 116 à une teneur en DX de 99%. Dans ce cadre, on soumettra le flux de dextrose 110 à une étape de séparation sélective des sucres 111, celle-ci pouvant consister en une (étape de) chromatographie couplée à une microfiltration et/ou déminéralisation (aucune particule ne pouvant entrer dans la chromatographie). Une étape de concentration préalable à la chromatographie peut également être mise en œuvre afin de pouvoir l'opérer dans les conditions optimales.

Les oligosaccharides 29 ainsi récupérés sont envoyés via une conduite 112 vers un réacteur 113, où ils sont mélangés au flux 21 de la conduite 121 pour y être transformés en un flux de dextrose fermentable 114.

Après cette dernière séparation 111, le flux de dextrose résultant 116 est envoyé au fermenteur pour y être soumis à une étape de fermentation 118, dans lequel on y ajoute un micro-organisme 26, producteur d'acide lactique 28 comme *Lactobacillus, Bacillus, Sporolactobacillus* ou autres dans des conditions opératoires bien connues pour réaliser cette opération.

Simultanément on envoie la fraction solide 21 récupérée des étapes de séparation successives (en utilisant successivement deux séparateurs 106 et 107) (via la conduite 121) et les oligosaccharides 29 via la conduite 112 dans un réacteur mélangeur 113 et on soumet le mélange à une hydrolyse pour les rendre fermentables. Cette hydrolyse est menée avec une grande quantité d'eau, généralement celle-ci représente de 50 à 80% du mélange, à une température comprise entre 140 et 180°C, généralement en présence d'acide fort et pendant une durée de 15 à 120 minutes. Suite à la réaction d'hydrolyse, on obtient un flux de dextrose fermentable 114 qui est envoyé via une conduite dans le fermenteur 120, dans lequel on y ajoute un micro-organisme 26 qui va transformer le second (flux ou) courant de dextrose en alcool, comme le butanol, ou l'éthanol pour être utilisé comme bioéthanol, et/ou en biogaz.

Pour la production d'alcool, les micro-organismes possibles seront des levures, comme *Saccharomyces, Schizosaccharomyces, Zymomonas mobilis* ou autres, ou des bactéries comme *Clostridia, Escherichia coli, Pseudomonas putida* ou autres.

Le système, utilisé dans la présente invention, pour produire simultanément en continu deux courants de dextrose au départ d'un broyage à sec de céréales, comprend:
- Un broyeur à grains 100 équipé pour effectuer un broyage à sec,
- Une cuve de mélange 102 (ou mélangeur) pour préparer un slurry 103 résultant du mélange du broyat 131 avec un courant d'eau,
- Un réacteur (pour effectuer une étape) de saccharification 104,
- Un filtre séparateur 106 pour donner deux flux de dextrose, l'un 108 contenant la matière liquide, l'autre 21 contenant la matière solide et résultant de deux étapes successives de séparation (en utilisant successivement deux séparateurs 106 et 107),
- Un séparateur 109 pour enlever l'huile du flux de dextrose 108, et récupérant un flux de dextrose purifié 110,
- Un système de séparation par chromatographie pour enlever les oligosaccharides 29 du flux de dextrose purifié 110 (le flux de dextrose purifié 110 résultant de la séparation effectuer dans le séparateur 109; ou autrement dit, le flux de dextrose purifié 110 récupéré de l'étape de séparation des huiles),
- Un fermenteur (pour effectuer une étape de fermentation 118) pour produire l'acide lactique 28,
- Un fermenteur 120 pour produire un alcool ou un biogaz.

Le procédé de la présente invention est également décrit au moyen d'un exemple ci-après.

### Exemple

On a broyé à sec, dans un broyeur approprié 100 des épis de maïs 20 à température ambiante et tous les éléments 131 qui résultent du broyage sont envoyés dans un mélangeur 102 pour y effectuer une étape de liquéfaction.

On introduit dans cette cuve agitée un mélange d'eau de recyclage du procédé ainsi qu'un apport d'eau fraiche. Bien que l'on puisse ajouter autant d'eau que l'on veut, on ajoute la quantité nécessaire pour rendre le mélange pompable.

On fait chauffer ce mélange à une température de 65°C durant 75 min en présence d'alpha amylase comme enzyme-catalyseur à raison de 0,01% en poids par rapport au mélange.

Le slurry résultant 103 est alors envoyé à la première étape de saccharification 104.

Ce slurry contient 33% de matière solide sèche.

On ajuste le pH du mélange à 4,5 par ajout d'un acide, en l'occurrence de l'acide sulfurique, et on a chauffé le mélange à 63°C pendant 3 heures, toujours en présence d'un catalyseur, en l'occurrence de l'alpha amylase utilisée à raison de 0,04% en poids.

On récupère le mélange et on le soumet à la seconde étape de saccharification 104 au cours de laquelle on réajuste le pH entre 4,1 et 4,3 tout en maintenant la température du mélange à 61°C pendant 5 heures.

On a obtenu ainsi un flux liquide 105 de dextrose brut ayant une teneur en dextrose de 90DE.

On fait passer ce flux de dextrose brut 105 sur un filtre 106 afin de séparer la partie solide 21 de la partie liquide 108; la partie solide 21 est récupérée pour être envoyée via une conduite 121 vers un réacteur 113.

Le flux liquide de dextrose 108 est alors soumis à une nouvelle séparation dans un séparateur 109 pour enlever l'huile surnageante qui résulte du broyage des grains de maïs.

Le flux de dextrose 110 résultant de cette séparation, ce flux 110 étant pratiquement exempt d'huile, est soumis à une séparation sélective des sucres 111 par chromatographie, ce qui permet de récupérer les oligosaccharides restants 29 et les envoyer via la conduite 112 au réacteur 113 avec les éléments insolubles et non fermentables 21.

Le flux de dextrose purifié (99DX) 116 résultant de cette dernière séparation 111 est envoyé au fermenteur 118 pour y effectuer une étape de fermentation pour y être transformé en acide lactique 28 après ajout d'un microorganisme 26 adéquat, ici un *Lactobacillus.*

D'autre part, le mélange qui arrive au réacteur 113 est soumis à une hydrolyse en présence d'un acide fort, ici l'acide chlorhydrique, à raison de 4% en poids d'acide par rapport au poids de matière solide présente.

On chauffe ensuite ce mélange à 150°C pendant 2 heures.

A la fin de l'opération, on obtient un flux de dextrose que l'on dirige vers le fermenteur 120 pour produire de l'éthanol en présence d'une levure adéquate, ici *Saccharomyces cervisiae.*

### Liste des symboles de références

- 20: céréales (comme par exemple grains et/ou épis de maïs)
- 21: flux (de dextrose) solide
- 25: huile(s)
- 26: microorganisme
- 27: enzyme
- 28: acide lactique
- 29: oligosaccharides (DPn)
- 100: broyeur
- 101: second produit de fermentation
- 102: mélangeur (ou cuve de mélange)
- 103: slurry
- 104: (étape de) saccharification
- 105: flux ou courant (de dextrose) brut
- 106: séparateur (comme e.g. filtre)
- 107: séparateur (comme e.g. filtre)
- 108: flux ou courant (de dextrose) liquide
- 109: séparateur
- 110: flux de dextrose purifié
- 111: (étape de) séparation sélective des sucres
- 112: conduite
- 113: réacteur d'hydrolyse
- 114: flux de dextrose fermentable
- 115: (étape de) filtration
- 116: flux de dextrose purifié
- 117: conduite
- 118: (étape de) fermentation
- 120: fermenteur
- 121: conduite
- 130: (étape d')affinage huiles
- 131: broyat (ou résultat du broyage à sec)

## Revendications

1. Procédé continu de production simultanée d'acide lactique et d'un second produit de fermentation au départ de céréales, qui comprend les étapes qui consistent à :
• Broyer à sec les grains et/ou les épis (20) de maïs dans un broyeur (100);
• Récupérer le broyat (131) et le mélanger dans un mélangeur (102) avec un courant d'eau afin de créer un slurry (103);
• Soumettre le slurry (103) à une saccharification (104) dans un réacteur et produire un courant de dextrose brut (105);
• Séparer dans un séparateur (106 et 107) le courant de dextrose brut (105) en deux flux, le premier constitué d'un flux liquide (108) et le second comprenant la partie solide (21);
• Enlever l'huile résultante du broyage et se trouvant dans le flux liquide (108) pour traitement ultérieur et récupérer un flux de dextrose purifié (110);
**Caractérisé en ce que** l'on :
• Soumet le flux de dextrose purifié (110) à une séparation sélective des sucres (111) et récupérer un flux purifié (116) avant de le soumettre à une fermentation (118); et récupérer d'autre part un flux riche en oligosaccharides (29)
• Soumet le flux purifié (116) à une fermentation (118) en présence d'un microorganisme (26) approprié pour produire de l'acide lactique (28);
• Récupère le flux de dextrose solide (21) via une conduite (121) contenant les produits difficilement fermentables, ainsi qu'un flux riche en oligosaccharides (29) via une conduite (112), pour les hydrolyser dans un réacteur (113) dans des conditions d'hydrolyse et les transformer en un flux de dextrose fermentable (114);
• Soumet le flux de dextrose fermentable (114) à une fermentation dans un fermenteur (120) pour produire un second produit de fermentation (101).

2. Procédé selon la revendication 1, **caractérisé en ce que** la séparation sélective des sucres (111) du flux de dextrose purifié (110) s'effectue par chromatographie.

3. Procédé selon la revendication 1, **caractérisé en ce que** l'hydrolyse dans le réacteur (113) s'effectue en présence d'un acide fort à une température comprise entre 140 et 180°C, pendant une période de 30 minutes à 2 heures.

4. Procédé selon la revendication 1, **caractérisé en ce que** le second produit de fermentation (101) est choisi parmi le groupe constitué par un alcool et/ou un biogaz.

5. Procédé selon la revendication **4, caractérisé en ce que** l'alcool est de l'éthanol ou du butanol.

6. Système pour produire simultanément en continu deux courants de dextrose au départ d'un broyage à sec de céréales, ce système comprenant :
• Un broyeur à grains (100) équipé pour un broyage à sec,
• Une cuve de mélange (102) pour préparer un slurry (103) résultant du mélange du broyat (131) avec un courant d'eau,
• Un réacteur de saccharification (104),
• Un séparateur (106) pour donner deux flux de dextrose, l'un (108) contenant la matière liquide, l'autre (21) la matière solide,
• Un séparateur (109) pour enlever l'huile du flux (108), et récupérant un flux de dextrose purifié (110),
• Un système de séparation par chromatographie pour enlever les oligosaccharides du flux de dextrose purifié (110),
• Un fermenteur pour produire l'acide lactique (28),
• Un fermenteur (120) pour produire l'alcool ou le biogaz.

## Patentansprüche

1. Kontinuierliches Verfahren zur gleichzeitigen Herstellung von Milchsäure und eines zweiten Fermentationsprodukts aus Zerealien, das die folgenden Schritte umfasst:
• trockenes Zerkleinern der Maiskörner und/oder -kolben (20) in einem Zerkleinerer (100);
• Rückgewinnen des Zerkleinerungsprodukts (131) und Mischen desselben in einem Mischer (102) mit einem Wasserstrom, um eine Aufschlämmung (103) zu erzeugen;
• Unterziehen der Aufschlämmung (103) einer Verzuckerung (104) in einem Reaktor und Produzieren eines Stroms roher Dextrose (105);
• Trennen des Stroms roher Dextrose (105) in einem Separator (106 und 107) in zwei Flüsse, wobei der erste aus einem flüssigen Fluss (108) besteht und der zweite den festen Teil (21) umfasst;
• Entfernen des Öls im Ergebnis des Zerkleinerns, das sich in dem flüssigen Fluss (108) befindet, für eine spätere Verarbeitung und Rückgewinnen eines gereinigten Dextroseflusses (110);
**dadurch gekennzeichnet, dass**:
• der gereinigte Dextrosefluss (110) einer selektiven Trennung der Zucker (111) unterzogen wird und ein gereinigter Fluss (116) zurückgewonnen wird, bevor er einer Fermentation (118) unterzogen wird; und zum anderen ein Fluss zurückgewonnen wird, der reich an Oligosacchariden (29) ist,
• der gereinigte Fluss (116) bei Anwesenheit eines für die Produktion von Milchsäure (28) geeigneten Mikroorganismus (26) einer Fermentation (118) unterzogen wird;
• der Fluss fester Dextrose (21), der die schwer fermentierbaren Produkte enthält, über eine Leitung (121) zurückgewonnen wird, sowie ein Fluss, der reich an Oligosacchariden (29) ist, über eine Leitung (112), um sie in einem Reaktor (113) unter Hydrolysebedingungen zu hydrolysieren und sie in einen Fluss fermentierbarer Dextrose (114) umzuwandeln;
• der Fluss fermentierbarer Dextrose (114) einer Fermentation in einem Fermenter (120) unterzogen wird, um ein zweite Fermentationsprodukt (101) zu produzieren.

2. Verfahren nach Anspruch 1, **dadurch gekennzeichnet, dass** die selektive Trennung der Zucker (111) des Flusses gereinigter Dextrose (110) durch Chromatographie erfolgt.

3. Verfahren nach Anspruch 1, **dadurch gekennzeichnet, dass** die Hydrolyse im Reaktor (113) bei Anwesenheit einer starken Säure bei einer Temperatur, die zwischen 140 und 180 °C liegt, während eines Zeitraums von 30 Minuten bis 2 Stunden erfolgt.

4. Verfahren nach Anspruch 1, **dadurch gekennzeichnet, dass** das zweite Fermentationsprodukt (101) aus der Gruppe ausgewählt ist, bestehend aus einem Alkohol und/oder einem Biogas.

5. Verfahren nach Anspruch 4, **dadurch gekennzeichnet, dass** der Alkohol Ethanol oder Butanol ist.

6. System, um gleichzeitig kontinuierlich zwei Dextroseströme ausgehend von einer trockenen Zerkleinerung von Zerealien zu produzieren, wobei dieses System umfasst:
• einen Kornzerkleinerer (100), der für eine trockene Zerkleinerung ausgestattet ist,
• einen Mischkessel (102) zur Herstellung einer Aufschlämmung (103) im Ergebnis des Mischens des Zerkleinerungsprodukts (131) mit einem Wasserstrom,
• einen Verzuckerungsreaktor (104),
• einen Separator (106) um zwei Dextroseflüsse zu erhalten, wobei einer (108) das flüssige Material und der andere (21) das feste Material enthält,
• einen Separator (109), um das Öl aus dem Fluss (108) zu entfernen und einen Fluss gereinigter Dextrose (110) zurückzugewinnen,
• ein Trennsystem durch Chromatographie, um die Oligosaccharide aus dem Fluss gereinigter Dextrose (110) zu entfernen,
• einen Fermenter, um die Milchsäure (28) zu produzieren,
• einen Fermenter (120), um den Alkohol oder das Biogas zu produzieren.

## Claims

1. Continuous method for the simultaneous production of lactic acid and of a second fermentation product from cereals, which comprises the following steps consisting of:
• dry milling the grains and/or the ears (20) of corn in a mill (100);
• recovering the milled product (131) and mixing it with a stream of water in a mixer (102) so as to create a slurry (103);
• subjecting the slurry (103) to a saccharification (104) in a reactor and producing a stream of crude dextrose (105);
• separating, in a separator (106 and 107), the stream of crude dextrose (105) into two flows, the first consisting of a liquid flow (108) and the second comprising the solid part (21);
• removing the oil resulting from the milling and present in the liquid flow (108) for subsequent treatment and recovering a purified dextrose flow (110);
**Characterized in that** it entails:
• subjecting the purified dextrose flow (110) to a selective separation of the sugars (111) and recovering a purified flow (116) before subjecting it to a fermentation (118); and on the other hand recovering a flow rich in oligosaccharides (29);
• subjecting the purified flow (116) to a fermentation (118) in the presence of a microorganism (26) appropriate for producing lactic acid (28);
• recovering the solid dextrose flow (21) via a pipe (121) containing the products which are difficult to ferment, as well as a flow rich in oligosaccharides (29) via a pipe (112), in order to hydrolyze them in a reactor (113) under hydrolysis conditions and transform them into a fermentable dextrose flow (114);
• subjecting the fermentable dextrose flow (114) to a fermentation in a fermenter (120) in order to produce a second fermentation product (101).

2. Method according to claim 1, **characterized in that** the selective separation of the sugars (111) from the purified dextrose flow (110) is done by chromatography.

3. Method according to claim 1, **characterized in that** the hydrolysis in the reactor (113) is done in the presence of a strong acid at a temperature of between 140 and 180°C, during a period from 30 minutes to 2 hours.

4. Method according to claim 1, **characterized in that** the second fermentation product (101) is chosen from the group consisiting of an alcohol and/or a biogas.

5. Method according to claim 4, **characterized in that** the alcohol is ethanol or butanol.

6. System for simultaneously continuously producing two streams of dextrose starting from dry milling of cereals, this system comprising:
• a grain mill (100) equipped for dry milling,
• a mixing vessel (102) for preparing a slurry (103) resulting from the mixing of the milled product (103) with a stream of water,
• a saccharification reactor (104),
• a separator (106) for yielding two dextrose flows, one (108) containing the liquid material, the other (21) the solid material,
• a separator (109) for removing the oil from the flow (108), and recovering a purified dextrose flow (110),
• a chromatographic separation system for removing the oligosaccharides from the purified dextrose flow (110),
• a fermenter for producing the lactic acid (20),
• a fermenter (120) for producing the alcohol or the biogas.
